(19) Europäisches Patentamt / European Patent Office / Office européen des brevets

(11) **EP 3 912 651 B1**

(12) **EUROPEAN PATENT SPECIFICATION**

(45) Date of publication and mention
of the grant of the patent:
**26.02.2025 Bulletin 2025/09**

(21) Application number: **19909670.2**

(22) Date of filing: **07.03.2019**

(51) International Patent Classification (IPC):
*A61L 24/04* (2006.01)     *A61L 24/00* (2006.01)
*A61L 26/00* (2006.01)

(52) Cooperative Patent Classification (CPC):
(C-Sets available)
**A61L 24/0042; A61L 24/043; A61L 26/0052;
A61L 26/009;** A61L 2400/04; A61L 2430/02
(Cont.)

(86) International application number:
**PCT/KR2019/002680**

(87) International publication number:
**WO 2020/149449 (23.07.2020 Gazette 2020/30)**

(54) **ABSORBENT BONE HEMOSTATIC MATERIAL COMPOSITION, AND BONE HEMOSTATIC MATERIAL MANUFACTURING METHOD USING SAME**

ABSORBIERENDE HÄMOSTATISCHE KNOCHENMATERIALZUSAMMENSETZUNG UND
VERFAHREN ZUR HERSTELLUNG VON HÄMOSTATISCHEM KNOCHENMATERIAL DAMIT

COMPOSITION DE MATÉRIAU HÉMOSTATIQUE OSSEUX ABSORBANT ET PROCÉDÉ DE
FABRICATION DE MATÉRIAU HÉMOSTATIQUE OSSEUX FAISANT APPEL À CELLE-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.01.2019 KR 20190005380**

(43) Date of publication of application:
**24.11.2021 Bulletin 2021/47**

(73) Proprietor: **Theracion Biomedical Co. Ltd.
Jungwon-gu
Seongnam-si, Gyeonggi-do 13201 (KR)**

(72) Inventors:
• **KIM, Eun Jin**
**Seongnam-si
Gyeonggi-do 13587 (KR)**
• **KIM, Sun Jong**
**Seongnam-si
Gyeonggi-do 13208 (KR)**
• **KIM, Byung Nam**
**Seongnam-si
Gyeonggi-do 13228 (KR)**

(74) Representative: **Klunker IP
Patentanwälte PartG mbB
Destouchesstraße 68
80796 München (DE)**

(56) References cited:
JP-B2- 5 770 666     KR-A- 20140 084 265
KR-A- 20160 112 511     KR-A- 20160 112 511
KR-B1- 101 494 299     US-A1- 2006 100 370
US-B1- 8 992 949

(52) Cooperative Patent Classification (CPC): (Cont.)

C-Sets
**A61L 24/043, C08L 71/02;
A61L 26/0052, C08L 71/02**

# EP 3 912 651 B1

**Description**

TECHNICAL FIELD

The present invention relates to an absorbent bone hemostatic material composition and an absorbent bone hemostatic material manufacturing method using the same, and more particularly, to an absorbent bone hemostatic material having an immediate hemostatic effect by providing a physical protective film in order to stop bone bleeding during surgery, and an absorbent bone hemostatic material manufacturing method using the same.

BACKGROUND ART

Bleeding is one of the obstacles that make surgery difficult, and stabilizing the bleeding is very important because a lot of blood loss may affect normal body functions. Hemostasis requires the use of a blood clotting agent, gauze, or a blood vessel occlusion device such as a hemostatic clamp. This type of hemostatic material utilizes the property of slowly activating the coagulation factors or narrowing the cut blood vessels. Numerous hemostatic materials have already been developed for soft tissue bleeding applications, but there is a limitation that cannot be applied to bone bleeding. This is because the blood flow hinders the stable formation of blood clots during bone hemorrhage, and the bone tissue itself maintains its structure through this condition. In the past, there have been many hemostatic materials that remain undecomposed in the body because non-biocompatible materials are included therein. As a result of many studies, it was confirmed that a hemostatic material had a negative effect on bone regeneration when components of the bone hemostatic material remained undecomposed.

Korean Patent Application Laid-Open No. 10-2014-0107429 relates to a hemostatic agent and a using method thereof. The hemostatic agent includes reverse micelles having an outer hydrophobic shell of suitable biocompatible hydrophobic components such as alkanes, and hydrophilic positively charged chitosan moieties enclosed within the hydrophobic shell, thereby attenuating or stopping bleeding. However, there is a limitation in that Korean Patent Application Laid-Open No. 10-2014-0107429 does not disclose the application to bone hemostasis and the immediate hemostasis by means of a physical protective film.

Korean Patent Application Laid-Open No. 10-2018-0055747 relates to a novel hemostatic agent including a mussel adhesive protein and a method for manufacturing an absorbent bone hemostatic agent using the same. Korean Patent Application Laid-Open No. 10-2018-0055747 does not disclose the application to bone hemostasis. A lot of ancillary materials such as nanofiber hemostatic dressings, hemostatic sponges and patches are required, and the hemostatic agent is difficult to decompose in vivo, and thus it is highly likely to hinder bone formation. Therefore, there is a need to develop a bone hemostatic agent that is quickly absorbed and decomposed without hindering bone formation and has an immediate hemostatic effect. KR 2016 0112511 A discloses a bioabsorbable composition for bone hemostasis comprising 20 to 50 parts by weight of poloxamer 407, 20 to 50 parts by weight of poloxamer 403 and 20 to 50 parts by weight of a polyethyleneglycol-polypropyleneglycol random copolymer.

Therefore, the present invention has been completed with the goal of developing a bone hemostatic agent that has an immediate hemostatic effect and minimally affects bone regeneration.

(Patent Literature 1) Korean Patent Application Laid-Open No. 10-2014-0107429 (2016. 09. 28.)
(Patent Literature 2) Korean Patent Application Laid-Open No. 10-2018-0055747 (2018. 05. 25.)

DESCRIPTION OF EMBODIMENTS

TECHNICAL PROBLEM

An object of the present invention is to solve the above-described problems. The invention is set out in the appended set of claims.

An object of the present invention is to provide an absorbent bone hemostatic material composition, which includes a minimal composition harmless to a human body and is quickly absorbed and decomposed in vivo, and thus does not hinder bone regeneration, and an absorbent bone hemostatic material manufacturing method using the same.

Another object of the present invention is to provide an absorbent bone hemostatic material composition having an immediate hemostatic effect and an absorbent bone hemostatic material manufacturing method using the same.

SOLUTION TO PROBLEM

In order to achieve the above-described objects of the present invention and achieve the characteristic effects of the present invention described below, the characteristic construction of the present invention is as follows.

An absorbent bone hemostatic material composition according to the present invention includes 50 to 70 parts by weight of polyethylene glycol-ran-propylene glycol based on 30 to 50 parts by weight of poloxamer.

The poloxamer has a weight average molecular weight of 7,000 to 10,000, and the polyethylene glycol-ran-propylene glycol has a weight average molecular weight of 6,000 to 12,000, and preferably 6,000 to 9,800.

The absorbent bone hemostatic material composition may have a hardness of 40 to 80 HS at a temperature of 20 to 40°C.

The absorbent bone hemostatic material composition may be biodegradable 80 to 100% within 10 to 50 hours in a phosphate buffer solution (PBS) of pH 7 to 8 under a temperature of 30 to 40°C.

The absorbent bone hemostatic material composition may have a hemostatic time of 0 to 10 seconds.

The absorbent bone hemostatic material composition may have an adhesive strength of 60 to 150 N.

The poloxamer may have a melting point of 40 to 100°C.

After the absorbent bone hemostatic material composition is used, a bone mineral density (BMD) may be restored to 0.5 to 0.6.

An absorbent bone hemostatic material may include the absorbent bone hemostatic material composition according to the present invention.

The absorbent bone hemostatic material may include at least one formulation selected from semi-solid and solid.

The absorbent bone hemostatic material may include a recess at a central portion.

On the other hand, the present invention provides an absorbent bone hemostatic material manufacturing method. Specifically, an absorbent bone hemostatic material manufacturing method including: an adding step of adding 50 to 70 parts by weight of polyethylene glycol-ran-propylene glycol into a reactor based on 30 to 50 parts by weight of poloxamer; a heating step of heating the poloxamer and the polyethylene glycol-ran-propylene glycol to 50 to 100°C; a stirring step of stirring the poloxamer and the polyethylene glycol-ran-propylene glycol; and a cooling step of cooling the poloxamer and the polyethylene glycol-ran-propylene glycol by pouring the poloxamer and the polyethylene glycol-ran-propylene glycol into a mold.

The cooling step may be performed for 10 to 60 minutes at a temperature of -30 to 10°C.

The poloxamer has a weight average molecular weight of 7,000 to 10,000, and the polyethylene glycol-ran-propylene glycol has a weight average molecular weight of 6,000 to 12,000, and preferably, 6,000 to 9,800.

ADVANTAGEOUS EFFECTS OF DISCLOSURE

An absorbent bone hemostatic material composition according to the present invention provides an effect of being quickly absorbed and decomposed in vivo.

An absorbent bone hemostatic material composition according to the present invention provides an immediate hemostatic effect.

An absorbent bone hemostatic material composition according to the present invention has an effect of being easily molded in a desired shape because the hardness thereof changes according to a temperature. Therefore, an effect of increasing adhesion is provided.

An absorbent bone hemostatic material composition according to the present invention includes a minimal material, is harmless to a human body, and is properly mixed to provide an effect that a finished product retains the efficacy of a raw material.

BRIEF DESCRIPTION OF DRAWINGS

FIG. 1 is a diagram illustrating an absorbent bone hemostatic material of the present invention.

FIG. 2 is a graph showing a degree of decomposition of an absorbent bone hemostatic material composition of the present invention according to a time.

FIG. 3 is a graph showing a hardness of the absorbent bone hemostatic material composition of the present invention according to a temperature.

FIG. 4 is a graph showing a time taken until hemostasis according to Example 1 and Comparative Example 2.

FIG. 5 is a spectroscopic graph showing physical properties of the absorbent bone hemostatic material composition and the absorbent bone hemostatic material.

FIG. 6 is photographs showing a degree of decomposition of the absorbent bone hemostatic material composition of the present invention and Comparative Example.

FIG. 7 is photographs showing a degree of bone regeneration of the absorbent bone hemostatic material composition of the present invention and Comparative Example.

FIG. 8 is photographs showing a degree of bioabsorption of the absorbent bone hemostatic material composition of the present invention and Comparative Example.

BEST MODE

The present invention will be described with reference to specific embodiments and the accompanying drawings. The embodiments will be described in detail in such a manner that the present invention may be carried out by those of ordinary skill in the art. It should be understood that various embodiments of the present invention are different, but need not be mutually exclusive. For example, certain shapes, structures, and features described herein may be implemented in other embodiments without departing from the scope of the present invention in connection with one embodiment. In addition, it should be understood that the locations or arrangement of individual components in the embodiments can be changed without departing from the scope of the present invention. Therefore, the following detailed description is not to be taken in a limiting sense, and the scope of the present invention is to be limited only by the appended claims, if properly explained. In the drawings, similar reference numerals refer to the same or similar functions throughout various aspects.

Hereinafter, embodiments of the present invention will be described with reference to the accompanying drawings, so that those of ordinary skill in the art can easily carry out the present invention.

An absorbent bone hemostatic material composition according to the present invention is a bone hemostatic material for immediate hemostasis. The present invention suggests an absorbent bone hemostatic material composition, which includes a minimal composition harmless to a human body and is quickly absorbed and decomposed in vivo, and thus does not hinder bone regeneration, and an absorbent bone hemostatic material manufacturing method using the same.

In the past, there was a problem that a hemostatic effect of a raw material could not be exhibited 100% in a finished product because the physical properties of the raw material and the physical properties of the finished product were different due to the reaction of each material during mixing. In addition, a lot of unnecessary materials are included so as to increase the hemostatic ability. In many cases, incompatible materials are included. Thus, bone regeneration is hindered. In particular, "BONE WAX" of Ethicon/J&J is applied to a cut surface to provide a physical protective film. However, "BONE WAX" is non-absorbent and remains on the treated region continuously. Therefore, there is a problem that inhibits the induction and growth of normal bone cells.

The absorbent bone hemostatic material composition according to the present invention includes a minimum of a bioabsorbent composition with high biocompatibility, and thus is almost harmless to a human body and quickly absorbed and decomposed. A physical protective film is provided by applying the absorbent bone hemostatic material composition to the bone surface at the time of fracture and bone cutting. Therefore, the absorbent bone hemostatic material composition has an immediate hemostatic effect and does not hinder normal bone regeneration.

The absorbent bone hemostatic material composition according to the present invention includes poloxamer and polyethylene glycol-ran-propylene glycol.

According to the present invention, the polyethylene glycol-ran-propylene glycol is included in an amount of 50 to 70 parts by weight based on 30 to 50 parts by weight of the poloxamer.

In addition, according to the present invention, the poloxamer has a weight average molecular weight of 7,000 to 10,000. When the poloxamer has a weight average molecular weight of less than 7,000 or greater than 10,000, it may be difficult to obtain the absorbent bone hemostatic material composition of the present invention that has an effect of immediate hemostasis and quick absorption and decomposition.

The polyethylene glycol-ran-propylene glycol has a weight average molecular weight of 6,000 to 12,000, and preferably 6,000 to 9,800. When the polyethylene glycol-ran-propylene glycol has a weight average molecular weight of less than 6,000 or greater than 12,000, it may be difficult to obtain the absorbent bone hemostatic material composition of the present invention that has an effect of immediate hemostasis and quick absorption and decomposition. Therefore, the above-described effect is more preferably provided in the range of 6,000 to 9,800.

According to the present invention, the polyethylene glycol-ran-propylene glycol is included in an amount of 50 to 70 parts by weight based on 30 to 50 parts by weight of the poloxamer. Preferably, the polyethylene glycol-ran-propylene glycol may be included in an amount of 61 parts by weight based on 39 parts by weight of the poloxamer. When the poloxamer is included in an amount of less than 30 parts by weight or greater than 50 parts by weight, the absorbent bone hemostatic material composition may not be mixed properly. Due to this, it may be difficult to obtain immediate bone hemostatic performance and satisfactory absorption or decomposition performance. When the polyethylene glycol-ran-propylene glycol is included in an amount of less than 50 parts by weight or greater than 70 parts by weight, a harmonious absorbent bone hemostatic material composition may not be prepared. Due to this, immediate hemostatic performance and the ability to be absorbed or decomposed in vivo may be reduced.

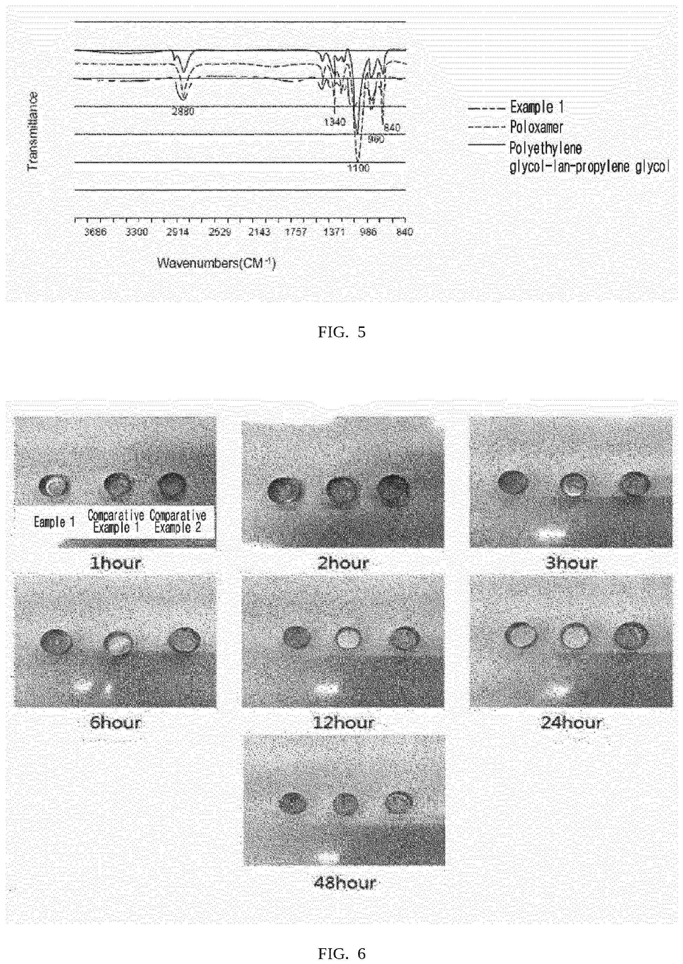

In addition, the poloxamer and the polyethylene glycol-lan-propylene glycol can be mixed properly. When the poloxamer and the polyethylene glycol-lan-propylene glycol are mixed properly, it is meaningful in that the characteristics of the raw materials can be maintained. Referring to the FT-IR result of FIG. 5, it can be confirmed that the absorbent bone hemostatic material composition and the bone hemostatic material according to the present invention are properly mixed with the raw materials, and thus the performance of the composition is equally shown even in the bone hemostatic material.

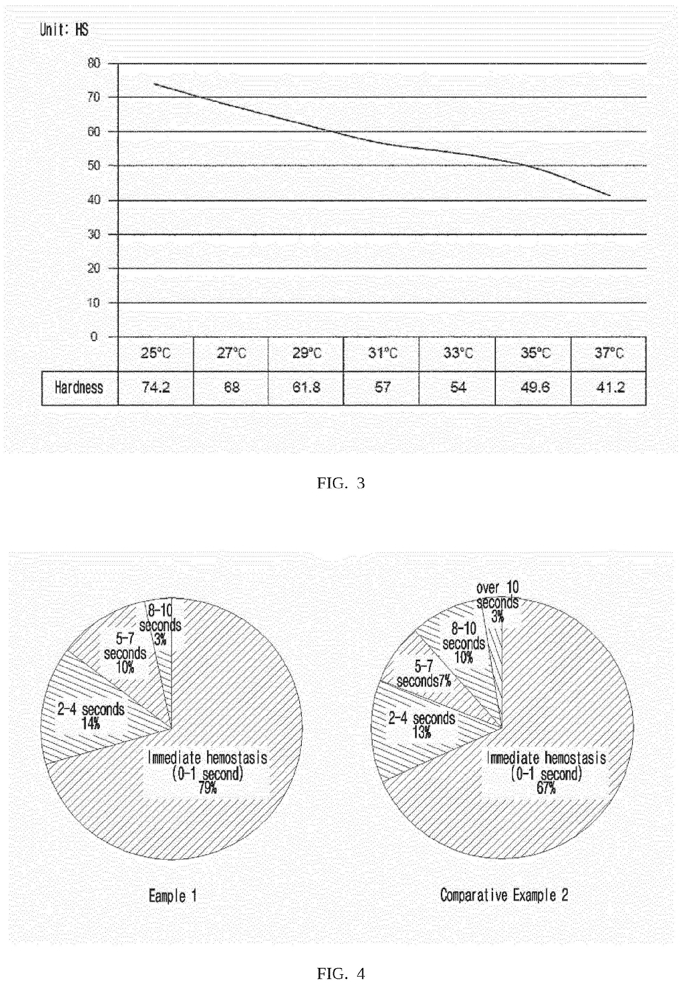

Referring to FIG. 3, the absorbent bone hemostatic material composition according to the present invention may have a hardness of 40 to 80 HS at a temperature of 20 to 40°C, preferably 60 to 80 HS at a temperature of 20 to 30°C and 40 to 60 HS at a temperature of 30 to 40°C. When the hardness is less than 40 HS at a temperature of 20 to 40°C, the absorbent bone hemostatic material composition may be so soft that it does not properly adhere to the bone. This may make it difficult to obtain satisfactory immediate hemostatic performance. In addition, when the hardness is greater than 80 HS at a temperature of 20 to 40°C, the absorbent bone hemostatic material composition may be so hard that it is difficult to mold the shape as desired. This may make it difficult to properly adhere to the bone and may make it difficult to obtain a satisfactory immediate hemostatic performance effect.

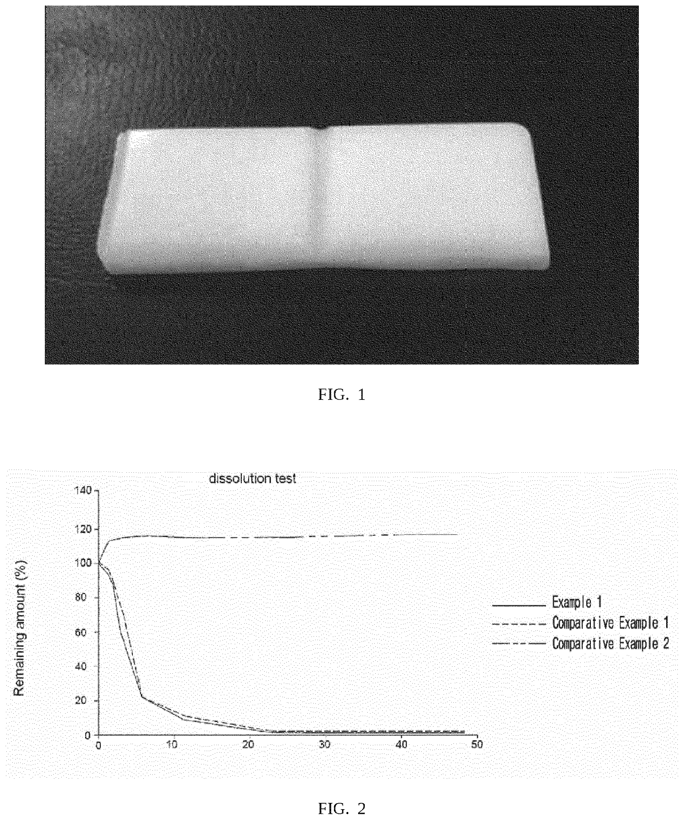

Referring to FIGS. 2 and 6, in the absorbent bone hemostatic material composition according to the present invention, biodegradation may be performed 80 to 100% within 10 to 50 hours in a phosphate buffer solution (PBS) of pH 7 to 8 under a temperature of 30 to 40°C. Preferably, biodegradation may be performed 90% within 12 hours, 96% within 24 hours, and more preferably 98% or more within 48 hours. When biodegradation is performed for more than 50 hours in a phosphate buffer solution (PBS) of pH 7 to 8 under a temperature of 30 to 40°C, an immune response of an immune system to foreign matters may occur, which may hinder bone formation.

In addition, in the absorbent bone hemostatic material composition according to the present invention, it may take 0 to 10 seconds for hemostasis. The bone hemostatic material, which is manufactured by including 50 to 70 parts by weight of the polyethylene glycol-ran-propylene glycol based on 30 to 50 parts by weight of the poloxamer, is more effective in the immediate hemostatic ability, compared with the conventional bone hemostatic material. The results thereof are shown in FIG. 4.

According to the present invention, the absorbent bone hemostatic material composition has an adhesive strength of 60 to 150 N. When the adhesive strength is less than 60 N, it is difficult to obtain a satisfactory immediate hemostatic effect at a bleeding site.

According to the present invention, the poloxamer has a melting point of 40 to 100°C. When the melting point is lower than 40°C, the bone hemostatic material is liquefied at body temperature, and thus it is difficult to obtain a hemostatic effect. When the melting point is higher than 100°C, it is necessary to perform a melting process with high heat in a manufacturing process, and thus the molecules of the poloxamer and the polyethylene glycol-lan-propylene glycol may not be arranged properly. Due to this, it may be difficult to obtain the absorbent bone hemostatic material composition having the immediate hemostatic performance and the quick absorption and decomposition performance.

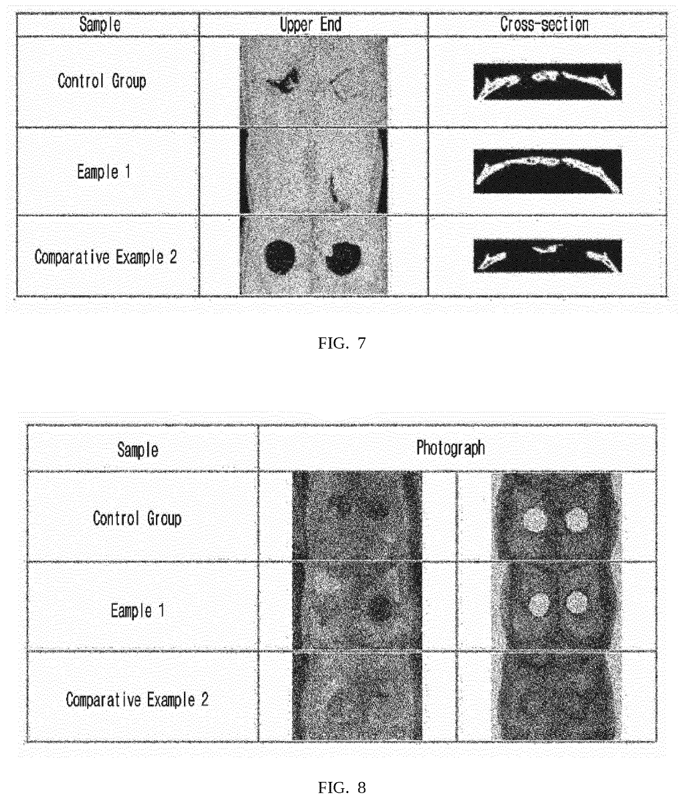

According to the present invention, after using the absorbent bone hemostatic material composition, a bone mineral density (BMD) may be 0.5 to 0.6, and preferably 0.51. Referring to FIG. 7, photographs comparing the bone formation ability of the absorbent bone hemostatic material composition according to the present invention and a comparative example can be confirmed.

According to the present invention, an absorbent bone hemostatic material includes the absorbent bone hemostatic material composition.

According to the present invention, the absorbent bone hemostatic material includes at least one formulation selected from semi-solid and solid.

In addition, the absorbent bone hemostatic material may include recesses in a central portion. As described above, since the recesses are formed in a line in the central portion, the absorbent bone hemostatic material can be easily removed.

On the other hand, according to the present invention, an absorbent bone hemostatic material manufacturing method using an absorbent bone hemostatic material composition is provided. The absorbent bone hemostatic material manufacturing method according to the present invention includes: an adding step of adding 50 to 70 parts by weight of polyethylene glycol-ran-propylene glycol into a reactor based on 30 to 50 parts by weight of poloxamer; a heating step of heating the poloxamer and the polyethylene glycol-ran-propylene glycol to 50 to 100°C; a stirring step of stirring the poloxamer and the polyethylene glycol-ran-propylene glycol; and a cooling step of cooling the poloxamer and the polyethylene glycol-ran-propylene glycol by pouring the poloxamer and the polyethylene glycol-ran-propylene glycol into a mold.

According to the present invention, in the heating step, the poloxamer and the polyethylene glycol-ran-propylene glycol may be preferably melted at a temperature of 80°C. When the heating temperature is lower than 50°C, the poloxamer and the polyethylene glycol-lan-propylene glycol may not be stirred properly. Due to this, it is difficult to obtain an absorbent bone hemostatic material composition having an immediate hemostatic effect. When the heating temperature is higher than 100°C, the molecules of the poloxamer and the polyethylene glycol-lan-propylene glycol may not be arranged properly. Due to this, it may be difficult to obtain an absorbent bone hemostatic material composition having immediate hemostatic performance and quick absorption and decomposition performance.

According to the present invention, the cooling step may be performed at a temperature of -30 to 10°C for 10 to 60 minutes. Preferably, the cooling step may be performed at a temperature of 4°C for 30 minutes. When the cooling step is performed at a temperature of lower than -30°C or higher than 10°C, it may be difficult to obtain the absorbent bone hemostatic material composition according to the present invention because the molecules are not arranged properly. In addition, even when the cooling step is performed for less than 10 minutes, it may be difficult to obtain the absorbent bone hemostatic material composition having satisfactory immediate hemostasis and quick absorption and decomposition effects.

In addition, according to the present invention, the poloxamer has a weight average molecular weight of 7,000 to 10,000. When the poloxamer has a weight average molecular weight of less than 7,000 or greater than 10,000, it may be difficult to obtain the absorbent bone hemostatic material composition of the present invention that has immediate hemostasis and quick absorption and decomposition effects.

According to the present invention, the polyethylene glycol-ran-propylene glycol has a weight average molecular weight of 6,000 to 12,000, and preferably 6,000 to 9,800. When the polyethylene glycol-ran-propylene glycol has a weight average molecular weight of less than 6,000 or greater than 12,000, it may be difficult to obtain the absorbent bone hemostatic material composition of the present invention that has an effect of immediate hemostasis and quick absorption and decomposition. Therefore, the above-described effect is more preferably provided in the range of 6,000 to 9,800.

According to the present invention, the polyethylene glycol-ran-propylene glycol is included in an amount of 50 to 70 parts by weight based on 30 to 50 parts by weight of the poloxamer. Preferably, the polyethylene glycol-ran-propylene glycol may be included in an amount of 61 parts by weight based on 39 parts by weight of the poloxamer. When the poloxamer is included in an amount of less than 30 parts by weight or greater than 50 parts by weight, the absorbent bone hemostatic material composition may not be mixed properly. Due to this, it may be difficult to obtain immediate bone hemostatic performance and satisfactory absorption or decomposition performance. When the polyethylene glycol-ran-propylene glycol is included in an amount of less than 50 parts by weight or greater than 70 parts by weight, a harmonious absorbent bone hemostatic material composition may not be prepared. Due to this, immediate hemostatic performance and the ability to be absorbed or decomposed in vivo may be reduced.

Referring to FIG. 3, the absorbent bone hemostatic material composition according to the present invention may have a hardness of 40 to 80 HS at a temperature of 20 to 40°C, preferably 60 to 80 HS at a temperature of 20 to 30°C and 40 to 60 HS at a temperature of 30 to 40°C. When the hardness is less than 40 HS at a temperature of 20 to 40°C, the absorbent bone hemostatic material composition may be too soft and thus may not be properly bonded to the bone. This may make it difficult to obtain satisfactory immediate hemostatic performance. In addition, when the hardness is greater than 80 HS at a temperature of 20 to 40°C, the absorbent bone hemostatic material composition may be so hard that it is difficult to mold the shape as desired. This may make it difficult to properly adhere to the bone and may make it difficult to obtain a satisfactory immediate hemostatic performance effect.

Referring to FIGS. 2 and 6, the absorbent bone hemostatic material composition according to the present invention is biodegradable 80 to 100% within 10 to 50 hours in a phosphate buffer solution (PBS) of pH 7 to 8 under a temperature of 30 to 40°C. Preferably, the absorbent bone hemostatic material composition is biodegradable 90% within 12 hours, 96% within 24 hours, and more preferably 98% or more within 48 hours. When biodegradation is performed for more than 50 hours in a phosphate buffer solution (PBS) of pH 7 to 8 under a temperature of 30 to 40°C, an immune response of an immune system to foreign matters may occur, which may hinder bone formation. Therefore, it can be confirmed that the case according to the present invention has a preferable biodegradability.

According to the present invention, the adhesive strength of the absorbent bone hemostatic material composition is 60 to 150 N. When the adhesive strength is less than 60 N, it is difficult to obtain a satisfactory immediate hemostatic effect.

Hereinafter, the structure and operation of the present invention will be described in more detail with reference to preferred examples of the present invention. However, these example are shown by way of illustration and should not be construed as limiting the present invention in any way.

Since contents not described herein can be sufficiently technically inferred by those of ordinary skill in the art, descriptions thereof will be omitted.

[Examples]

<Example 1>

39 parts by weight of poloxamer and 61 parts by weight of polyethylene glycol-ran-propylene glycol were added to a reactor. The added poloxamer and polyethylene glycol-ran-propylene glycol were melted at a temperature of 80°C. After sufficient stirring, the poloxamer and the polyethylene glycol-ran-propylene glycol were poured into a mold, placed into a cooler, and cooled at a temperature of 4°C for 30 minutes to prepare a solidified absorbent bone hemostatic material (TAB-WAX).

<Comparative Example 1>

Comparative Example 1 was an Ostene hemostatic material from Ceremed/Baxter.

<Comparative Example 2>

Comparative Example 2 was a BONEWAX hemostatic material from Ethicon/J&J.

<Experimental Example 1> Dissolution Test

The absorbent bone hemostatic material of the present invention manufactured in Example 1 has to serve as a physical barrier at a treated site for a certain time so as to stop bone bleeding, and gradually melt and disappear from the treated site after a certain time for normal bone healing. For this test, the following experiment was performed.

For the experiment, 1 g of the absorbent bone hemostatic material composition manufactured in Example 1 was precisely weighed in 20 ml of Phosphate buffer solution (PBS) (pH 7.4) at 37°C and placed into a conical tube. The absorbent bone hemostatic material composition was taken out from a shaking bath (37°C, 60 RPM) at a set time, and the amount of the bone hemostatic material that was decomposed and reduced was measured as a weight loss rate. Table 1 shows the results of calculating the degree of decomposition according to Equation 1 below. In addition, the results thereof are shown in FIG. 6.

[Equation 1]

$$\text{Degree of decomposition} = \frac{\text{(Initial sample weight)} - \text{(Sample weight after 48 hours)}}{\text{(Initial sample weight)}} \times 100$$

[Table 1]

| Time (h) | Biodegradability (%) | |
|---|---|---|
| | Example 1 | Comparative Example 2 |
| 0 | 0.0 | 0.0 |
| 1 | 3.3 | -13.1 |
| 2 | 10.3 | -14.7 |
| 3 | 37.8 | -15.6 |
| 6 | 75.4 | -16.2 |
| 12 | 89.2 | -15.0 |
| 24 | 97.0 | -15.7 |
| 48 | 97.8 | -17.7 |

As described in Table 1, referring to FIGS. 2 and 6, it was confirmed that Example 1 (TAB-WAX), which is the absorbent bone hemostatic material according to the present invention, achieved a biodegradability of 97.8% within 48 hours, compared with Comparative Example 2 (BONEWAX). On the other hand, Comparative Example 2 was not decomposed over time and showed a negative value. Therefore, it can be seen that the sample weight increased after 48 hours, and an immune system recognized it as a foreign matter in a human body, causing a reaction such as an inflammatory reaction.

<Experimental Example 2> Hardness measurement

An appropriate amount of the absorbent bone hemostatic material of the present invention manufactured in Example 1 should be cut out, kneaded by hand, and then applied to a site to be treated. Therefore, hardness directly related to kneading properties was measured for each temperature, and the results thereof are shown in Table 2 and the graph of FIG. 3.

[Table 2]

| Temperature (°C) | 25 | 27 | 29 | 31 | 33 | 35 | 37 |
|---|---|---|---|---|---|---|---|
| Hardness (HS) | 74.2 | 68 | 61.8 | 57 | 54 | 49.6 | 41.2 |

As shown in Table 2, it can be expected that, when kneaded by hand, the absorbent bone hemostatic material according to the present invention can be kneaded more conveniently due to the body temperature of the user's hand.

<Experimental Example 3> Evaluation of immediate hemostatic ability

After Example 1 and Comparative Example 2 were applied to a lesion site, the time taken until hemostasis was measured. The time of hemostasis was evaluated and recorded in seconds from 0 seconds to 10 seconds through photography and was divided into five sections (section 1: 0-1 seconds [immediate], section 2: 2-4 seconds, section 3: 5-7 seconds, section 4: 7-9 seconds, and section 5: 10 seconds or more). This is shown in FIG. 4.

When comparing Example 1 (TAB-WAX) coated with the sample with Comparative Example 2 (BONEWAX), Example 1 was 73% and Comparative Example 2 was 67%. Therefore, it was confirmed that the absorbent bone hemostatic material according to the present invention had excellent immediate hemostatic performance. There was no subject in which hemostasis was delayed for more than 10 seconds, whereas a rate of 3% was confirmed in Comparative Example 2.

<Experimental Example 4> Evaluation of bioabsorption

Referring to FIG. 8, the first-row photograph of autopsy photographs for each group is a photograph immediately shown after skin and periosteum were sequentially incised, and the second-row photograph is a photograph taken against a white background by cutting a rat skull with a size of about 15 cm x 15 cm around a defect.

As a result of visual evaluation, in the case of Example 1, there was no formulation remaining from the third day after surgery, but in the case of Comparative Example 2 (BONEWAX), it was confirmed that the formulation remained as it is until 12 weeks.

<Experimental Example 5> Evaluation of bone formation ability

In order to evaluate the degree of bone formation ability (bone healing), a bone mineral density (BMD) and a bone volume ratio (BVR) were measured by analyzing 3D images of a rat skull through microCT.

Referring to FIG. 7, as a result of microCT analysis, in the bone volume ratio value, bone volumes of a control group and a group using Example 1 were increased to a similar degree as time passed for 3 weeks, 6 weeks, and 12 weeks. At the twelfth week, the BMD value became the same value of 0.51. Both the control group and Example 1 showed excellent bone forming ability.

**Claims**

1. An absorbent bone hemostatic material composition comprising 50 to 70 parts by weight of polyethylene glycol-ran-propylene glycol based on 30 to 50 parts by weight of poloxamer,

    wherein the poloxamer has a weight average molecular weight of 7,000 to 10,000,
    **characterized in that** the polyethylene glycol-ran-propylene glycol has a weight average molecular weight of 6,000 to 12,000.

2. The absorbent bone hemostatic material composition of claim 1, wherein the absorbent bone hemostatic material composition has a hardness of 40 to 80 HS at a temperature of 20 to 40°C.

3. The absorbent bone hemostatic material composition of claim 1, wherein the absorbent bone hemostatic material composition is biodegradable 80 to 100% within 10 to 50 hours in a phosphate buffer solution (PBS) of pH 7 to 8 under a temperature of 30 to 40°C.

4. The absorbent bone hemostatic material composition of claim 1, wherein the absorbent bone hemostatic material composition has a hemostatic time of 0 to 10 seconds.

5. The absorbent bone hemostatic material composition of claim 1, wherein the absorbent bone hemostatic material composition has an adhesive strength of 60 to 150 N.

6. The absorbent bone hemostatic material composition of claim 1, wherein the poloxamer has a melting point of 40 to 100°C.

7. The absorbent bone hemostatic material composition of claim 1, wherein, after the absorbent bone hemostatic material composition is used, a bone mineral density (BMD) is restored to 0.5 to 0.6.

8. An absorbent bone hemostatic material comprising the absorbent bone hemostatic material composition of claims 1 to 7.

9. The absorbent bone hemostatic material of claim 8, wherein the absorbent bone hemostatic material comprises at least one formulation selected from semi-solid and solid.

10. The absorbent bone hemostatic material of claim 9, wherein the absorbent bone hemostatic material comprises a recess at a central portion.

11. An absorbent bone hemostatic material manufacturing method comprising:

an adding step of adding 50 to 70 parts by weight of polyethylene glycol-ran-propylene glycol into a reactor based on 30 to 50 parts by weight of poloxamer;
a heating step of heating the poloxamer and the polyethylene glycol-ran-propylene glycol to 50 to 100°C;
a stirring step of stirring the poloxamer and the polyethylene glycol-ran-propylene glycol; and
a cooling step of cooling the poloxamer and the polyethylene glycol-ran-propylene glycol by pouring the poloxamer and the polyethylene glycol-ran-propylene glycol into a mold,
wherein the poloxamer has a weight average molecular weight of 7,000 to 10,000,**characterized in that** the polyethylene glycol-ran-propylene glycol has a weight average molecular weight of 6,000 to 12,000.

12. The absorbent bone hemostatic material manufacturing method of claim 11, wherein the cooling step is performed for 10 to 60 minutes at a temperature of -30 to 10°C.

**Patentansprüche**

1. Saugfähige, blutstillende Knochenmaterialzusammensetzung, umfassend 50 bis 70 Gewichtsteile Polyethylenglycol-ran-propylenglycol, bezogen auf 30 bis 50 Gewichtsteile Poloxamer, wobei das Poloxamer ein durchschnittliches Molekulargewicht von 7.000 bis 10.000 aufweist, **dadurch gekennzeichnet, dass** das Polyethylenglykol-ran-Propylenglykol ein gewichtsdurchschnittliches Molekulargewicht von 6.000 bis 12.000 aufweist.

2. Saugfähige, blutstillende Knochenmaterialzusammensetzung nach Anspruch 1, wobei die Zusammensetzung des absorbierenden, blutstillenden Knochenmaterials eine Härte von 40 bis 80 HS bei einer Temperatur von 20 bis 40 °C aufweist.

3. Saugfähige, blutstillende Knochenmaterialzusammensetzung nach Anspruch 1, wobei die absorbierende, blutstillende Knochenmaterialzusammensetzung innerhalb von 10 bis 50 Stunden in einer Phosphatpufferlösung (PBS) mit einem pH-Wert von 7 bis 8 bei einer Temperatur von 30 bis 40 °C zu 80 bis 100 % biologisch abbaubar ist.

4. Saugfähige, blutstillende Knochenmaterialzusammensetzung nach Anspruch 1, wobei die absorbierende, blutstillende Knochenmaterialzusammensetzung eine Blutstillungszeit von 0 bis 10 Sekunden aufweist.

5. Saugfähige, blutstillende Knochenmaterialzusammensetzung nach Anspruch 1, wobei die absorbierende, blutstillende Knochenmaterialzusammensetzung eine Haftfestigkeit von 60 bis 150 N aufweist.

6. Saugfähige, blutstillende Knochenmaterialzusammensetzung nach Anspruch 1, wobei das Poloxamer einen Schmelzpunkt von 40 bis 100 °C aufweist.

7. Saugfähige, blutstillende Knochenmaterialzusammensetzung nach Anspruch 1, wobei nach der Verwendung der absorbierenden, blutstillenden Knochenmaterialzusammensetzung eine Knochenmineraldichte (BMD) von 0,5 bis 0,6 wiederhergestellt wird.

8.  Saugfähiges, blutstillendes Knochenmaterial das die saugfähige, blutstillende Knochenmaterialzusammensetzung nach den Ansprüchen 1 bis 7 umfasst.

9.  Saugfähiges, blutstillendes Knochenmaterial nach Anspruch 8, wobei das saugfähige, blutstillende Knochenmaterial mindestens eine Formulierung umfasst, die aus halbfest und fest ausgewählt ist.

10. Saugfähiges, blutstillendes Knochenmaterial nach Anspruch 9, wobei das saugfähige, blutstillende Knochenmaterial eine Aussparung an einem zentralen Abschnitt umfasst.

11. Verfahren zur Herstellung eines absorbierenden, blutstillenden Knochenmaterials, umfassend: einen Zugabeschritt, bei dem 50 bis 70 Gewichtsteile Polyethylenglycol-ran-propylenglycol in einen Reaktor gegeben werden, bezogen auf 30 bis 50 Gewichtsteile Poloxamer, einen Erhitzungsschritt, bei dem das Poloxamer und das Polyethylenglykol-ran-Propylenglykol auf 50 bis 100 °C, einen Rührschritt, bei dem das Poloxamer und das Polyethylenglykol-ran-Propylenglykol gerührt werden, und einen Kühlschritt, bei dem das Poloxamer und das Polyethylenglykol-ran-Propylenglykol gekühlt werden, indem das Poloxamer und das Polyethylenglykol-ran-propylenglykol in eine Form gegossen wird, wobei das Poloxamer ein gewichtsmittleres Molekulargewicht von 7000 bis 10000 aufweist, **dadurch gekennzeichnet, dass** das Polyethylenglykol-ran-propylenglykol ein gewichtsmittleres Molekulargewicht von 6000 bis 12000 aufweist.

12. Verfahren zur Herstellung eines absorbierenden, blutstillenden Knochenmaterials nach Anspruch 11, wobei der Kühlschritt 10 bis 60 Minuten lang bei einer Temperatur von -30 bis 10 °C durchgeführt wird.

**Revendications**

1.  Composition de matériau hémostatique osseux absorbant, comprenant de 50 à 70 parties en poids de polyéthylène glycol-ran-propylène glycol sur la base de 30 à 50 parties en poids de poloxamère,

    dans laquelle le poloxamère présente un poids moléculaire moyen en poids allant de 7000 à 10 000, **caractérisée en ce que** le polyéthylène glycol-ran-propylène glycol présente un poids moléculaire moyen en poids allant de 6000 à 12 000.

2.  Composition de matériau hémostatique osseux absorbant selon la revendication 1, dans laquelle la composition de matériau hémostatique osseux absorbant présente une dureté allant de 40 à 80 HS à une température allant de 20 à 40 °C.

3.  Composition de matériau hémostatique osseux absorbant selon la revendication 1, dans laquelle la composition de matériau hémostatique osseux absorbant est biodégradable de 80 % à 100 % dans les limites de 10 à 50 heures dans une solution tamponnée au phosphate (PBS, Phosphate Buffer Solution) d'un pH de 7 à 8, à une température allant de 30 à 40 °C.

4.  Composition de matériau hémostatique osseux absorbant selon la revendication 1, dans laquelle la composition de matériau hémostatique osseux absorbant présente un temps hémostatique allant de 0 à 10 secondes.

5.  Composition de matériau hémostatique osseux absorbant selon la revendication 1, dans laquelle la composition de matériau hémostatique osseux absorbant présente une résistance d'adhérence de 60 à 150 N.

6.  Composition de matériau hémostatique osseux absorbant selon la revendication 1, dans laquelle le poloxamère présente un point de fusion allant de 40 à 100 °C.

7.  Composition de matériau hémostatique osseux absorbant selon la revendication 1, dans laquelle après utilisation de la composition de matériau hémostatique osseux absorbant, une densité minérale osseuse (BMD) est restaurée jusqu'à 0,5 à 0,6.

8.  Matériau hémostatique osseux absorbant comprenant la composition de matériau hémostatique osseux absorbant selon l'une quelconque des revendications 1 à 7.

9. Matériau hémostatique osseux absorbant selon la revendication 8, dans lequel le matériau hémostatique osseux absorbant comprend au moins une formulation sélectionnée parmi des formulations semi-solides et solides.

10. Matériau hémostatique osseux absorbant selon la revendication 9, dans lequel le matériau hémostatique osseux absorbant comprend un retrait sur une portion centrale.

11. Procédé de fabrication de matériau hémostatique osseux absorbant, comprenant les étapes suivantes :

   une étape d'addition pour ajouter 50 à 70 parties en poids du polyéthylène glycol-ran-propylène glycol dans un réacteur sur la base de 30 à 50 parties en poids de poloxamère ;
   une étape de chauffage pour chauffer le poloxamère et le polyéthylène glycol-ran-propylène glycol à 50 jusqu'à 100 °C ;
   une étape de mélangeage pour mélanger le poloxamère et le polyéthylène glycol-ran-propylène glycol, et
   une étape de refroidissement pour refroidir le poloxamère et le polyéthylène glycol-ran-propylène glycol, en versant le poloxamère et le polyéthylène glycol-ran-propylène glycol dans un moule,
   dans lequel le poloxamère présente un poids moléculaire moyen en poids allant de 7000 à 10 000,
   **caractérisé en ce que** le polyéthylène glycol-ran-propylène glycol présente un poids moléculaire moyen en poids allant de 6000 à 12 000.

12. Procédé de fabrication de matériau hémostatique osseux absorbant selon la revendication 11, dans lequel l'étape de refroidissement est réalisée pendant 10 à 60 minutes à une température allant de -30 à 10 °C.

FIG. 1

FIG. 2

Unit: HS

| | 25°C | 27°C | 29°C | 31°C | 33°C | 35°C | 37°C |
|---|---|---|---|---|---|---|---|
| Hardness | 74.2 | 68 | 61.8 | 57 | 54 | 49.6 | 41.2 |

FIG. 3

Eample 1

Comparative Example 2

FIG. 4

FIG. 5

FIG. 6

| Sample | Upper End | Cross-section |
|---|---|---|
| Control Group | | |
| Eample 1 | | |
| Comparative Example 2 | | |

FIG. 7

| Sample | Photograph |
|---|---|
| Control Group | |
| Eample 1 | |
| Comparative Example 2 | |

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020140107429
- KR 1020180055747
- KR 20160112511 A